# FASCICULE DE BREVET EUROPEEN

(11) **EP 2 265 229 B1**
(45) Date de publication et mention de la délivrance du brevet: **08.05.2019**
(21) Numéro de dépôt: 09726457.6
(22) Date de dépôt: 12.03.2009
(51) Int. Cl.: A61F 9/00, A61M 5/32

(54) **DISPOSITIF D'INIECTION DANS L'OEIL**
AUGENINJEKTIONSVORRICHTUNG
EYE INJECTION DEVICE

(30) Priorité: 14.03.2008 FR 0801415
(43) Date de publication de la demande: 29.12.2010
(73) Titulaire: Institut National de la Santé et de la Recherche Médicale (INSERM), 75654 Paris Cedex 13 (FR)
(72) Inventeur: TOUCHARD, Elodie, F-75006 Paris (FR); KOWALCZUK, Laura, F-75006 Paris (FR); BEHAR- COHEN, Francine, F-75006 Paris (FR)
(74) Mandataire: Nony
(86) Numéro de dépôt international: PCT/FR2009/000260
(87) Numéro de publication internationale: WO 2009/122030

(56) Documents cités:
- WO-A-00/07530
- WO-A-03/070297
- WO-A-2006/052557
- WO-A-2007/052730
- US-A1- 2006 253 124

## Description

### Domaine technique

La présente invention se rapporte à un dispositif destiné à l'injection d'un produit dans un oeil et/ou à une électroporation.

### Etat de la technique

WO 2006/123248 décrit un dispositif destiné à l'administration par électroporation d'un produit. Dans le mode de réalisation représenté sur la figure 16, le dispositif est conformé pour pouvoir être appliqué à la surface de l'oeil et comporte une aiguille permettant une injection de produit. La mise en place de ce dispositif à la surface de l'oeil est délicate. En outre, le positionnement est relativement imprécis. Enfin, une erreur de positionnement nécessite de multiplier les piqûres dans l'oeil pour repositionner le dispositif.

WO 00/07530, WO 2007/052730 et WO 2006/052557 décrivent des dispositifs d'injection. Ces dispositifs ne comportent pas une aiguille d'injection fixée sur un support destiné à prendre appui sur la surface de l'oeil ou ne présentent pas une marque de repérage apte à prendre appui sur le rebord de la cornée d'un oeil avant tout contact de l'aiguille d'injection avec l'oeil, puis à être maintenue dans cette position d'appui pendant la phase de pénétration de ladite aiguille d'injection dans l'oeil, ladite pénétration résultant d'une rotation du support autour de la marque de repérage.

### Résumé de l'invention

L'invention est définie par l'objet de la revendication 1 et des revendications dépendantes.

Un objet de l'invention est de fournir un dispositif d'injection permettant de résoudre, au moins partiellement, un ou plusieurs des problèmes susmentionnés. Il est décrit un dispositif d'injection destiné à l'injection d'un produit dans un oeil (c'est-à-dire dans les milieux oculaires ou dans un tissu de l'oeil), le dispositif comportant :
- une aiguille d'injection,
- un support sur lequel l'aiguille d'injection est fixée ou apte à guider un coulissement de l'aiguille d'injection,
le dispositif comportant une marque de repérage disposée de manière à pouvoir être mise en contact avec une région d'appui déterminée de la surface de l'oeil avant tout contact de l'aiguille d'injection avec ladite surface, puis être maintenue en contact avec ladite région d'appui pendant une phase de pénétration de ladite aiguille d'injection à travers ladite surface de l'oeil.

Comme cela apparaîtra plus en détail dans la suite de la description, la marque de repérage permet à l'utilisateur de positionner, avec une précision remarquable, le dispositif d'injection à la surface de l'oeil avant toute pénétration de l'aiguille d'injection à travers la surface de ce dernier. Le risque d'erreur est donc réduit, voire sensiblement nul.

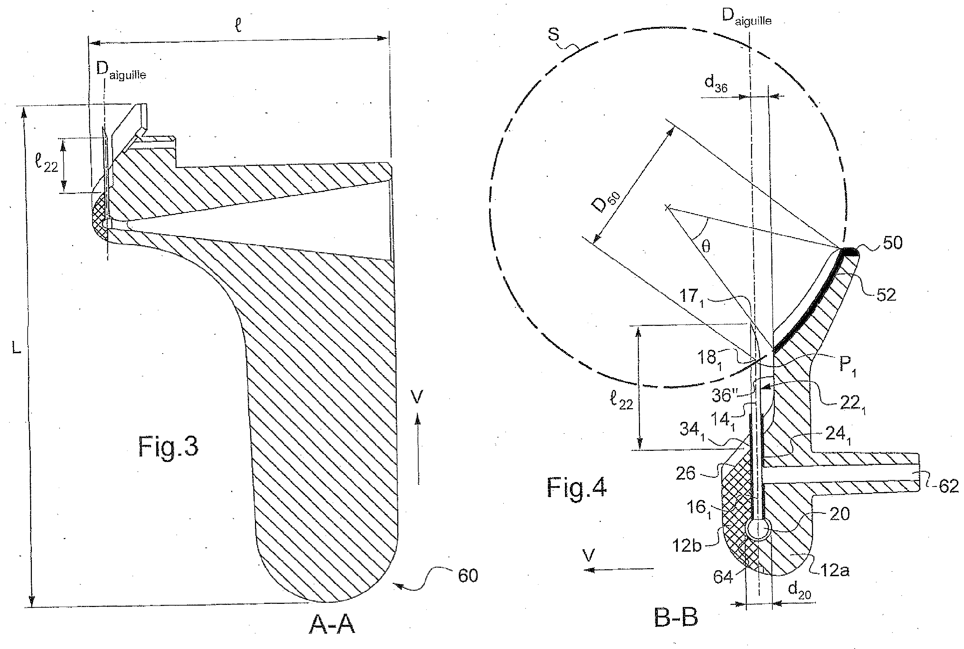

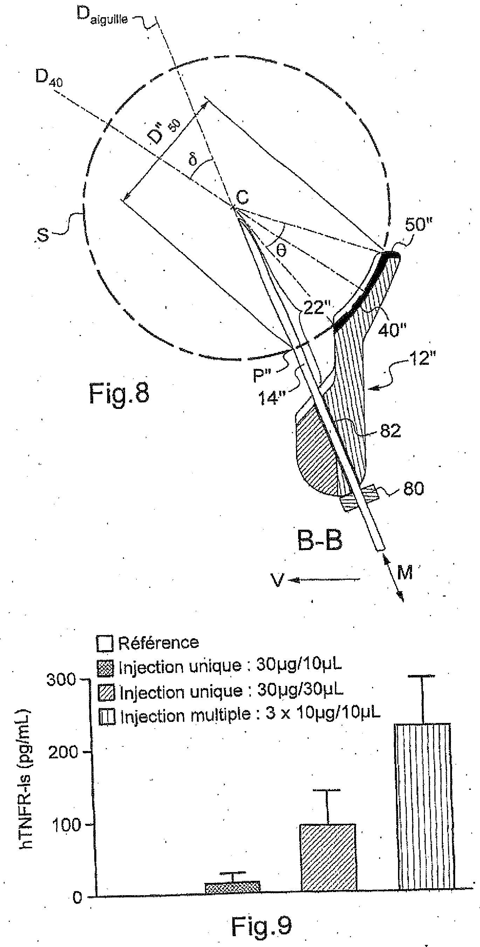

Dans un mode de réalisation, notamment destiné à une injection dans une cavité vitréenne, la marque de repérage est disposée de manière que, pendant ladite phase de pénétration de l'aiguille d'injection à travers la surface de l'oeil, la distance entre la région d'appui de la marque de repérage sur l'oeil et le point de pénétration de l'aiguille dans l'oeil (correspondant à la distance D₅₀ sur la figure 4 et à la distance D₅₀" sur la figure 8) soit supérieure à 2 mm, supérieure à 3 mm, voire supérieure à 3,5 mm et/ou inférieure à 6 mm, inférieure à 5 mm, voire inférieure à 4,5 mm. Une distance de 4 mm est bien adaptée.

Notamment lorsque le dispositif est destiné à une injection dans un muscle ciliaire, cette distance peut être supérieure à 1 mm, supérieur à 1,5 mm et/ou inférieure à 4 mm, inférieure à 3 mm, voire inférieure à 2, 5 mm. Une distance de 2 mm est bien adaptée.

En outre, la marque de repérage est disposée de manière à pouvoir être maintenue en contact avec la région d'appui pendant au moins une partie, de préférence pendant la totalité, de la pénétration de l'aiguille d'injection à travers la surface de l'oeil. L'utilisateur bénéficie ainsi d'une aide précieuse pour contrôler et supprimer tout déplacement inopportun du support pendant la phase de pénétration de l'aiguille d'injection dans l'oeil. En particulier, dans un mode de réalisation, la marque de repérage est conformée pour coopérer avec la région d'appui de manière à limiter, voire supprimer, la possibilité d'un déplacement de la marque de repérage par rapport à la région d'appui pendant la phase de pénétration de l'aiguille d'injection.

Ladite région d'appui comprend une partie du rebord de la cornée, c'est-à-dire de l'épaulement de transition entre la cornée et la sclérotique, appelé « limbe ».

Une marque de repérage assurant un appui sur plus de 5 mm, plus de 7 mm, voire plus de 9 mm sur ce rebord, permet avantageusement une bonne stabilisation du dispositif pendant la phase de pénétration.

Ladite aiguille d'injection peut être conformée de manière que son extrémité distale atteigne le muscle ciliaire ou la cavité vitréenne, ou l'espace sous rétinien, ou la rétine, ou l'épithélium pigmentaire de la rétine, ou la cornée épithelium ou stroma ou endothélium ou transcornéen), ou encore des muscles péri-oculaires. De préférence, des moyens sont prévus pour que cette extrémité distale ne puisse être enfoncée au-delà de ces organes lors de la phase de pénétration, la région d'appui étant au moins une partie du rebord de la cornée de l'oeil.

La marque de repérage et l'aiguille d'injection peuvent être configurées de manière que l'angle entre la direction de pénétration et la direction perpendiculaire à la surface de l'oeil au point de pénétration soit inférieur à 20°, voire 15°, 10° ou 5° pendant toute la phase de pénétration. La longueur de l'aiguille d'injection introduite dans l'oeil est alors avantageusement réduite.

### Aiguille

Le diamètre extérieur de l'aiguille d'injection peut être compris entre 0,2 et 0,4 mm.

La partie en porte-à-faux de l'aiguille d'injection, c'est-à-dire faisant saillie du support, peut être supérieure à 2 mm ou supérieure à 3 mm, voire supérieure à 3,3 mm et/ou inférieure à 5 mm, voire inférieure à 4 mm, ou inférieure à 3,7 mm.

La pointe, ou « extrémité distale », de l'aiguille d'injection peut être biseautée afin de faciliter son enfoncement dans l'oeil. L'aiguille d'injection peut déboucher axialement et/ou radialement au niveau de son extrémité distale. De préférence, l'aiguille d'injection débouche axialement. De préférence encore, elle est effilée, c'est-à-dire conique selon l'axe de l'aiguille, et débouche axialement.

La forme de l'aiguille d'injection n'est pas limitative.

L'aiguille d'injection peut être sensiblement rectiligne. Elle peut également s'étendre suivant un arc de cercle centré sur un point de la marque de repérage, notamment pour faciliter son enfoncement par rotation du support autour de la région d'appui.

Pour fixer efficacement l'aiguille d'injection, la base de cette dernière peut être prise en sandwich entre une partie principale du support et une pièce de blocage fixée sur ladite partie principale de support.

Le dispositif peut comporter plusieurs aiguilles d'injection et en particulier comporter trois, voire plus de trois, aiguilles d'injection, identiques ou différentes.

Les aiguilles d'injection peuvent présenter une longueur totale identique quelle que soit l'aiguille d'injection considérée.

Les parties en porte-à-faux des aiguilles d'injection peuvent être identiques ou différentes. Elles peuvent être en particulier de la même longueur.

Les aiguilles d'injection peuvent être toutes fixées de la même manière sur le support.

Dans une variante de l'invention, une ou plusieurs des aiguilles d'injection ne sont pas fixées sur le support mais peuvent coulisser sur ce dernier lors de la phase de pénétration. Ces aiguilles peuvent être amovibles ou être montées solidaires du support, c'est-à-dire de manière à rester liées au support.

Dans ce dernier cas, elles peuvent avantageusement être rétractables dans ledit support.

Dans un mode de réalisation, les aiguilles d'injection sont écartées les unes des autres, deux à deux, d'une distance supérieure à 3 mm, ou supérieure à 3,5 mm, voire supérieure à 4 mm et/ou inférieure à 6 mm, ou inférieure 5,5 mm, voire inférieure à 5 mm.

Les aiguilles d'injection peuvent être sensiblement parallèles et/ou coplanaires.

Les extrémités distales des aiguilles d'injection peuvent s'étendre suivant une ligne, par exemple présentant un plan de symétrie. Cette ligne peut, par exemple, être rectiligne. Elle peut aussi être courbe, par exemple suivre un arc de cercle. En particulier, lorsque le support comporte une face de butée sphérique, comme décrit ci-après, cette ligne peut présenter le même rayon de courbure que cette face de butée, ou être concentrique à cette dernière.

Les extrémités distales des aiguilles d'injection peuvent ne pas appartenir à un même plan perpendiculaire à la direction locale desdites aiguilles d'injection au niveau de leurs extrémités distales.

Dans un mode de réalisation, les extrémités proximales des aiguilles d'injection, c'est-à-dire les extrémités opposées aux extrémités distales, débouchent dans un canal de distribution commun à l'ensemble des aiguilles d'injection, le canal de distribution étant par exemple ménagé dans le support. Le support peut alors présenter des moyens de connexion destinés à mettre en communication de fluide le canal de distribution et une source du produit à injecter.

Dans un mode de réalisation, le dispositif comporte encore des moyens permettant de boucher sélectivement ou simultanément une ou plusieurs, et de préférence toutes, les lumières de la ou des aiguilles d'injection, par exemple au niveau des extrémités proximales ou du canal de distribution.

Ces moyens peuvent notamment comprendre un ou plusieurs bouchons aptes à boucher chacun une seule ou plusieurs aiguilles d'injection.

Dans un mode de réalisation, chaque aiguille d'injection peut être alimentée indépendamment des autres. Par exemple, chaque aiguille peut être connectée à une tubulure individuelle. Avantageusement, il est ainsi possible d'injecter différents principes actifs par les différentes aiguilles d'injection du dispositif.

Dans ce mode de réalisation, un clampage ou un pincement d'une tubulure permet de couper l'alimentation de l'aiguille d'injection associée.

Toutes les tubulures peuvent aussi être connectées à une tubulure principale dont le clampage ou le pincement conduit à couper l'alimentation de toutes les tubulures simultanément.

### Support

Le support peut servir à la manipulation du dispositif. Le support peut notamment comporter une poignée permettant, par exemple une préhension du dispositif d'injection entre un pouce et un index d'une main. La manipulation du dispositif en est facilitée. Cette poignée peut notamment s'étendre sensiblement suivant un plan général parallèle à la ou aux aiguilles d'injection.

La longueur du support peut être supérieure à 10 mm ou 30 mm et/ou être inférieure à 100 mm, 50 mm ou 40 mm. Sa largeur transversale peut être supérieure à 10 mm ou 15 mm et/ou inférieure à 30 mm, ou inférieure à 25 mm.

Le support peut être en un matériau transparent afin qu'il soit possible pour l'utilisateur d'observer plus facilement le point de pénétration de l'aiguille.

Dans un mode de réalisation, le support est adapté de manière à pouvoir maintenir les paupières de l'oeil en position ouverte pendant la phase de pénétration de l'aiguille d'injection. A cet effet, il peut notamment comporter des rebords latéraux, par exemple d'une hauteur supérieure à 0,5 mm, 1 mm, 2 mm, voire 3 mm. Pour améliorer le maintien d'une paupière sur un rebord, le support peut encore comporter une protubérance apte à venir se loger entre la surface de l'oeil et la paupière à écarter lorsque cette paupière s'appuie sur le rebord.

Avantageusement, le support fait ainsi office d'écarteur des paupières, ce qui assure une sécurité optimale et évite tout risque de fermeture brutale des paupières pendant l'injection.

### Moyens de butée

Le dispositif peut comporter des moyens de butée aptes à empêcher une pénétration de la ou des aiguilles d'injection dans l'oeil au-delà d'une position déterminée, dite « position de service ».

Les moyens de butée peuvent comprendre des « moyens de butée du support » aptes à limiter le mouvement du support lors de la phase de pénétration. En particulier, lorsque le support est mis en mouvement pendant la phase de pénétration, ces moyens de butée du support permettent d'empêcher la poursuite de ce mouvement au-delà d'une position déterminée, par exemple au-delà d'une position où une face de butée du support épouse la surface de l'oeil. Les moyens de butée du support sont ainsi conformés pour autoriser un positionnement du support sur l'oeil dans une position déterminée, ou « position de butée du support ». Une position de butée du support peut être en particulier obtenue lorsque la marque de repérage prend appui sur le limbe de l'oeil et qu'une face de butée du support épouse la sclérotique de l'oeil. Lorsque la ou les aiguilles d'injection sont fixées sur le support, la position de service correspond à la position de butée du support.

Dans le mode de réalisation où une aiguille d'injection est montée coulissante sur le support, les moyens de butée peuvent également comprendre des moyens de butée « d'aiguille », conformés pour limiter la longueur de ladite aiguille pouvant faire saillie du support depuis l'extrémité distale de l'aiguille, c'est-à-dire la longueur de la partie en porte-à-faux de l'aiguille. L'aiguille d'injection peut par exemple comporter une surépaisseur pouvant venir en butée sur le support. On appelle « position de butée d'aiguille », la position de l'aiguille correspondant à une longueur maximale de la partie en porte-à-faux de l'aiguille, c'est-à-dire la position dans laquelle les moyens de butée d'aiguille sont actifs. Dans ce mode de réalisation, la position de service correspond de préférence à une configuration dans laquelle le support est dans une position de butée du support et l'aiguille d'injection est dans une position de butée d'aiguille.

De préférence, les moyens de butée sont conformés de manière à limiter à moins de 20 mm, notamment pour une injection dans la rétine, moins de 7 mm, voire à moins de 3 mm, notamment pour une injection dans le muscle ciliaire, voire à moins de 1 mm, voire à moins de 0,7 mm, la longueur de la ou des aiguilles d'injection pouvant être enfoncée dans l'oeil lors d'une phase de pénétration de l'aiguille d'injection, ou « longueur d'enfoncement maximal ». De préférence la longueur d'enfoncement maximal est supérieure à 0,5mm et inférieure à 14 mm.

Les moyens de butée du support comportent de préférence une face de butée destinée à entrer en contact avec la surface de l'oeil et présentant de préférence une forme complémentaire à cette surface, en particulier à la sclérotique de l'oeil, de manière à pouvoir, dans la position de butée du support, épouser la forme dudit oeil et ainsi assurer un appui fiable.

La face de butée peut comporter des moyens anti-dérapants aptes à limiter le glissement du dispositif dans la position de butée du support.

Dans un mode de réalisation, la face de butée présente une aire supérieure à 4 mm² et/ou inférieure à 21 mm². La face de butée peut être pleine ou être localement percée par des orifices.

La face de butée peut présenter une forme sphérique, le rayon de courbure R pouvant être notamment supérieur à 9 mm, 10 mm ou 11 mm et/ou inférieur à 14 mm, inférieur 13 mm, ou inférieur à 12 mm. Un rayon de courbure de 11,75 mm est bien adapté.

De préférence, la face de butée s'étend latéralement, c'est-à-dire suivant sa plus grande dimension en vue de face, sur un secteur angulaire α supérieur à 45°, de préférence supérieur à 60°, voire 70°, 75°, ou même 80° et/ou inférieur à 135°, de préférence inférieur à 120°, voire 100°, 95° ou même 90°.

De préférence, la face de butée s'étend en hauteur, c'est-à-dire suivant sa plus petite dimension en vue de face, sur un secteur angulaire θ supérieur à 15°, voire supérieur à 25°, ou même 30° et/ou inférieur à 55°, voire inférieur à 45°, ou même 40°.

De préférence, la face de butée ne s'étend pas sur plus d'un quadrant d'une demi-sphère.

En vue de face, la face de butée peut présenter un contour sensiblement parallélépipédique, par exemple rectangulaire.

La face de butée peut ainsi présenter deux grands côtés et deux petits côtés. Les grands côtés peuvent en particulier former avec les petits côtés des angles arrondis.

La longueur des petits côtés peut être supérieure ou égale à 2mm et/ou inférieure à 3mm. La longueur des grands côtés peut être supérieure à 2 mm et/ou inférieure à 7 mm.

De préférence, la face de butée est agencée pour n'entrer en contact avec la surface de l'oeil qu'à la fin de la phase de pénétration de l'aiguille d'injection.

De préférence également, dans la position de butée du support, le support ne prend appui sur la surface de l'oeil que par l'intermédiaire de la face de butée.

Dans le cas où les aiguilles d'injection sont coplanaires, le plan des aiguilles peut être sensiblement parallèle aux grands cotés.

L'axe principal de la face de butée destinée à entrer en contact avec l'oeil peut être incliné par rapport à la direction d'une ou plusieurs des aiguilles ou, si ces aiguilles sont coplanaires, par rapport au plan dans lequel elles s'étendent, d'un angle δ supérieur à 20° et/ou inférieur à 85°.

Notamment dans le cas où le dispositif d'injection est destiné à une injection dans un muscle ciliaire, l'angle δ peut être supérieur à 30°, 40° 50° et/ou inférieur à 80°, 70°, 60°. Un angle d'environ 55° est bien adapté.

Dans un mode de réalisation, notamment dans le cas où le dispositif d'injection est destiné à une injection au coeur de la cavité vitréenne, l'angle δ peut être déterminé de manière que la ou les aiguilles d'injection s'étendent sensiblement suivant une direction passant par le centre C d'une enveloppe sphérique S suivant laquelle la face de butée s'étend. Il peut être alors avantageux que, dans la position de butée du support, au moins une des aiguilles d'injection passe par le centre de l'oeil.

Notamment lorsque le dispositif est destiné à une injection dans la cavité vitréenne, l'angle δ peut être supérieur à 20°, supérieur à 30° et/ou inférieur à 45°, voire inférieur à 40°, un angle d'environ 35 ° étant bien adapté.

Dans un mode de réalisation, le dispositif comporte une face de butée s'étendant sensiblement suivant une enveloppe sphérique et la marque de repérage s'étend selon une ligne de repérage suivant un arc de cercle dont l'axe passe par le centre de ladite enveloppe sphérique.

L'enveloppe sphérique et l'arc de cercle peuvent en particulier être déterminés de manière que, dans la position de service, la face de butée soit complètement en appui sur la sclérotique de l'oeil et la marque de repérage soit complètement en appui sur le rebord de la cornée.

Dans un mode de réalisation, le dispositif comporte une face de butée s'étendant sensiblement suivant une enveloppe sphérique, la marque de repérage s'étend selon une ligne de repérage en arc de cercle, au moins une aiguille pénètre dans l'enveloppe sphérique par un point de pénétration et, dans un plan passant par le centre de ladite enveloppe sphérique, par ledit point de pénétration et coupant perpendiculairement ledit arc de cercle, la distance entre ledit point de pénétration et le point dudit arc de cercle par lequel ledit plan coupe ledit arc de cercle est
(1) supérieure à 1 mm, de préférence supérieure à 1,5 mm, de préférence encore supérieure à 1,8 mm et inférieure à 3 mm, de préférence inférieure à 1,5 mm, de préférence encore inférieure à 1,2 mm, ladite aiguille s'étendant à l'intérieur de ladite enveloppe sphérique jusqu'à une profondeur supérieure à 0,5 mm, de préférence supérieure à 0,6 mm, de préférence encore supérieure à 0,8 mm et inférieure à 1,5 mm, de préférence inférieure à 1,3 mm, de préférence encore inférieure à 1,2 mm ; ou
(2) supérieure à 2 mm, de préférence supérieure à 3 mm et inférieure à 4,5 mm, de préférence inférieure à 4 mm, voire inférieure à 3,5 mm, ladite aiguille s'étendant à l'intérieur de ladite enveloppe sphérique jusqu'à une profondeur supérieure à 6 mm, de préférence supérieure à 8 mm et inférieure à 15 mm, de préférence inférieure à 13 mm.

De préférence toutes les aiguilles respectent simultanément la condition (1), ce mode de réalisation étant particulièrement bien adapté pour une injection dans le muscle ciliaire de l'oeil, ou la condition (2), ce mode de réalisation étant particulièrement bien adapté pour une injection dans le corps vitréen.

La profondeur est la distance minimale entre la surface de l'enveloppe sphérique et l'extrémité distale de l'aiguille considérée, c'est-à-dire habituellement la distance entre cette surface et cette extrémité distale mesurée sur un rayon de l'enveloppe sphérique S.

De préférence, toutes les aiguilles s'étendent à l'intérieur de l'enveloppe sphérique jusqu'à atteindre une profondeur identique.

De préférence, la ou les aiguilles d'injection sont insérées sur le support du même côté que les moyens de butée, par rapport à la marque de repérage.

Dans un mode de réalisation, la ou les aiguilles d'injection ne traversent pas la face de butée. Elles peuvent en particulier émerger d'une « face d'insertion », qui, dans la position de butée du support, n'est pas en contact avec l'oeil. L'écartement entre l'oeil et la face d'insertion permet avantageusement à l'utilisateur d'observer la surface de l'oeil à proximité du point de pénétration de l'aiguille. Il facilite également le positionnement du support.

La face d'insertion peut être en retrait par rapport à la face de butée et être, par exemple, conformée de manière que, dans la position de butée du support, elle soit écartée de la surface de l'oeil d'une distance supérieure à 1 mm et/ou inférieure à 1cm.

Les moyens de butée du support peuvent être fixés sur le support ou être venus de matière avec lui.

Les moyens de butée du support sont de préférence définis de manière que, dans la position de butée du support, la ou les aiguilles soient inclinées suivant une direction prédéfinie, et, de préférence, immobilisées par rapport à la surface de l'oeil.

Lorsque le support comporte des moyens de guidage d'une aiguille d'injection aptes à guider un coulissement de celle-ci, des moyens de butée d'aiguille peuvent être avantageusement prévus afin de limiter la longueur de l'aiguille d'injection faisant saillie en porte-à-faux, dans la position sortie de cette aiguille d'injection.

Bien entendu, un dispositif selon l'invention peut comporter une ou plusieurs aiguilles d'injection fixées sur le support et une ou plusieurs aiguilles d'injection aptes à coulisser sur ledit support.

### Marque de repérage

Le dispositif comporte une ligne de repérage apte à épouser la surface de l'oeil, par exemple le rebord de la cornée. Elle suit un arc de cercle d'un rayon supérieur à 5 mm, de préférence 6 mm et inférieur à 8 mm, de préférence 7 mm, un rayon de 6,58 mm étant bien adapté.

La ligne de repérage suivant un arc de cercle, ledit point d'insertion est de préférence à l'extérieur de la surface sphérique virtuelle dont un grand cercle inclut la ligne de repérage.

De préférence, lorsque les moyens de butée comportent une face de butée sensiblement sphérique, le point d'insertion de l'aiguille dans le support, et de préférence, tous les points d'insertion de toutes les aiguilles, est (sont) à l'extérieur de la surface sphérique virtuelle sur laquelle s'étend la face de butée.

La longueur de la ligne de repérage qui est en contact avec la surface de l'oeil peut dépendre du degré de pénétration de la ou des aiguilles d'injection. Par exemple, au début de la phase de pénétration, la ligne de repérage peut être en contact par un ou plusieurs points, voire une ou plusieurs fractions de cette ligne, avec la surface de l'oeil, puis, au cours de la pénétration, la nature de ce contact peut évoluer.

La longueur de la marque de repérage est suffisamment faible pour que, compte tenu de la souplesse de l'oeil, la marque de repérage puisse rester, sur toute sa longueur, en contact avec le rebord de la cornée de l'oeil, pendant toute la phase de pénétration. Pour faciliter le maintien en contact de la marque de repérage avec le rebord de la cornée pendant toute la phase de pénétration de l'aiguille d'injection, la marque de repérage est conformée pour n'assurer un appui que sur une longueur inférieure à 20 mm, de préférence à 17 mm, voire inférieure à 15 mm, de ce rebord.

La marque de repérage est fixée sur le support, sur les moyens de butée ou sur tout autre organe rigidement solidarisé au support. Elle doit cependant être immobile par rapport au support, au moins pendant la phase de pénétration, de préférence en permanence.

Dans un exemple ne formant pas partie de l'invention, la marque de repérage peut être portée, par exemple, par une ou plusieurs aiguilles d'injection ou, le cas échéant, une ou plusieurs électrodes, invasive(s) ou non invasive(s).

Dans un mode de réalisation, la marque de repérage est portée par des moyens de butée, par exemple par un grand côté d'une face de butée, et en particulier, dans le cas d'une face de butée de forme sensiblement rectangulaire, vue de face, par le grand coté le plus éloigné des aiguilles d'injection.

Dans un exemple ne formant pas partie de l'invention, la marque de repérage peut être portée, voire être constituée, par un bord d'une électrode non invasive.

La marque de repérage étant destinée à être mise en contact avec la surface d'un oeil, elle présente de préférence une surface lisse, et, de préférence encore, dépourvue d'aspérités, notamment sous forme de pointe ou d'arête vive, susceptible d'endommager la surface de l'oeil lors de la phase de pénétration. Elle peut être en un matériau souple, c'est-à-dire non agressif vis-à-vis la surface de l'oeil, par exemple des polymères en silicone, en éponge conductrice, notamment en éponge synthétique, en polyester, en polyorthoester, en polyméthacrylate de méthyle ou en tout autre polymère médical grade souple et, de préférence, être translucide.

La marque de repérage peut en particulier être constituée par une bande en un matériau souple d'une largeur supérieure à 1,5 mm et/ou inférieure à 5 mm, s'étendant par exemple le long d'un bord, par exemple sur toute la longueur, de moyens de butée ou d'une électrode non invasive.

La marque de repérage peut en particulier être constituée par un bourrelet en silicone ou en mousse. Avantageusement, le risque de lésion du limbe (bord de la cornée) est ainsi réduit.

La face de butée et/ou la marque de repérage peuvent également comporter des moyens de ventouse aptes à se fixer sur la surface de l'oeil dans la position de butée du support. Ces moyens peuvent être passifs (mousse exposant une multitude de cellules ouvertes, ventouses), c'est-à-dire se fixer d'eux-mêmes sur la surface de l'oeil par simple pression contre cette surface. Ils peuvent également être actifs, c'est-à-dire nécessiter une aspiration.

Avantageusement, ces moyens de ventouse contribuent à stabiliser le support dans la position de butée du support.

La marque de repérage et/ou les moyens de butée du support peuvent être constitués ou être revêtus au moins partiellement par un matériau anti-dérapant apte à limiter le glissement à la surface de l'oeil.

Comme expliqué ci-dessus, la marque de repérage est disposée de manière à pouvoir rester en contact avec la surface de l'oeil pendant la pénétration de la ou des aiguilles d'injection à travers cette surface.

Dans un mode de réalisation, la marque de repérage est conformée de manière à guider la pénétration de la ou des aiguilles d'injection dans l'oeil tout en restant en contact avec la surface de l'oeil. En particulier la marque de repérage peut servir de point de rotation à la ou aux aiguilles d'injection lors de ladite pénétration. Une augmentation, au cours de ladite pénétration, de la surface de la marque de repérage en contact avec la surface de l'oeil peut également améliorer la précision de cette pénétration.

### Moyens de guidage

Une aiguille d'injection peut être fixée sur le support, c'est-à-dire être en permanence immobilisée sur le support, ou, en variante, être montée mobile sur le support, de manière amovible ou non.

A cet effet, le support peut présenter des moyens de guidage, par exemple sous la forme d'un rail de coulissement permettant un coulissement guidé de l'aiguille d'injection sur le support. Le rail de coulissement peut être notamment un orifice traversant de section complémentaire à celle de l'aiguille d'injection qu'il est destiné à recevoir. Les moyens de guidage peuvent guider l'aiguille d'injection dans des mouvements suivant la courbe de l'aiguille. Dans le cas d'une aiguille rectiligne, l'aiguille d'injection est ainsi guidée suivant son axe. Une rotation autour de son axe reste possible.

En variante, le rail de guidage peut également empêcher une telle rotation.

La portée du rail de guidage, c'est-à-dire la distance entre les deux points les plus éloignés du rail de guidage, peut être supérieure à 2 mm, supérieure à 3 mm, supérieure à 4 mm, voire supérieure à 5 mm. Une grande portée permet avantageusement un guidage efficace.

L'aiguille d'injection peut être amovible, c'est-à-dire peut éventuellement être totalement désolidarisée du support.

L'invention concerne donc également un dispositif selon l'invention mais ne comportant pas nécessairement d'aiguille d'injection. En particulier, l'invention propose un support comportant une marque de repérage disposée de manière à pouvoir être mise en contact, suivant une ligne de contact d'une longueur d'au moins 2 mm, voire d'au moins 3 mm, 5 mm, 7 mm, 9 mm, voire 11 mm, avec le limbe d'un oeil, puis à pouvoir être maintenue en position d'appui sur le limbe tandis qu'une aiguille d'injection et/ou une électrode invasive se déplace, guidée par le support.

Le support peut présenter une ou plusieurs des caractéristiques optionnelles ou nécessaires des supports des dispositifs selon l'invention. En particulier, il peut comporter une face de butée de forme complémentaire à la surface de l'oeil.

Dans un mode de réalisation, le support est configuré de manière que
- la marque de repérage puisse être disposée en contact, suivant ladite ligne de contact, avec le limbe de l'oeil, et
- la face de butée puisse être disposée en appui sur la surface de l'oeil, sans qu'une aiguille d'injection ou une électrode invasive ait dû entrer en contact avec l'oeil.

### Electrodes

Dans un mode de réalisation, le dispositif comporte au moins une électrode, invasive ou non invasive. De préférence, le dispositif comporte un jeu d'électrodes constitué de premier et deuxième ensembles d'électrodes destinés à être connectés électriquement à des première et deuxième bornes, respectivement, d'un générateur électrique.

Le jeu d'électrodes peut notamment comporter des électrodes disposées de manière à assurer une électroporation facilitant le transfert du produit sortant de la ou des aiguilles d'injection.

Dans un mode de réalisation particulier, au moins un orifice d'éjection d'une aiguille d'injection du dispositif débouche entre une électrode du premier ensemble d'électrodes et une électrode du deuxième ensemble d'électrodes de manière que du produit puisse sortir de l'aiguille d'injection dans une zone qui puisse être couverte par un champ électrique généré par lesdites électrodes.

Dans un mode de réalisation, une ou plusieurs des électrodes invasives, voire toutes les électrodes invasives, sont isolées électriquement sur au moins une partie de leur longueur par exemple au moyen d'un revêtement isolant. En particulier, la partie isolée électriquement de la partie en porte-à-faux peut s'étendre depuis le support sur une longueur comprise entre 0,5 mm et 3 mm, par exemple d'environ 1 mm, notamment lorsque l'électrode invasive est destinée à être piquée dans un muscle ciliaire, ou comprise entre 10 mm et 14 mm ou entre 11 mm et 13 mm, par exemple d'environ 12 mm, notamment lorsque l'électrode invasive est destinée à être piquée dans une cavité vitréenne, ou encore de manière plus générale sur une longueur comprise entre 0,5 mm voire 1 mm et 30 mm. Toute la partie en porte-à-faux peut être isolée électriquement.

La partie d'une électrode invasive en contact avec le support, dite « partie insérée», peut présenter une longueur comprise entre 10 et 20 mm, par exemple d'environ 15 mm. De préférence, la partie insérée est isolée électriquement du support.

Une aiguille d'injection peut être, au moins en partie, voire totalement, en un matériau conducteur électriquement de manière à pouvoir constituer une électrode invasive. Lorsque le dispositif comporte plusieurs aiguilles d'injection, une ou plusieurs de ces aiguilles d'injection, voire toutes ces aiguilles d'injection, peuvent constituer des électrodes invasives. Les premier et deuxième ensembles d'électrodes peuvent par exemple être constitués par des aiguilles d'injection.

Une ou plusieurs des caractéristiques optionnelles décrites ci-dessus pour la ou les aiguilles d'injection du dispositif peuvent être appliquées à une ou plusieurs électrodes invasives du dispositif, que ces dernières soient des aiguilles d'injection ou non.

Dans un mode de réalisation, le dispositif comporte trois aiguilles d'injection en communication de fluide et appartenant à un même ensemble d'électrodes.

L'autre ensemble d'électrode peut être constitué, par exemple, par une ou plusieurs électrodes non invasives, appartenant ou non au dispositif.

De préférence, le dispositif comporte une ou plusieurs électrodes non invasives. Une électrode non invasive peut être souple ou rigide.

Dans un mode de réalisation, le dispositif comporte une électrode non invasive rigide présentant un élément conducteur électriquement destiné à entrer en contact avec la surface de l'oeil et de préférence conformé de manière à pouvoir épouser cette dernière.

De préférence, l'élément conducteur comporte, voire est constitué, par un revêtement en un matériau conducteur électriquement, recouvrant au moins partiellement, de préférence sensiblement en totalité, une face de butée telle que décrite précédemment.

Le revêtement conducteur peut s'étendre par exemple sur toute la face de butée à l'exception d'un bord destiné à servir de marque de repérage et constitué de préférence par un matériau souple, comme défini précédemment. L'élément conducteur peut également comporter, voire être constitué par, une pluralité de plots connectés électriquement entre eux et disposés, de préférence régulièrement, à la surface de contact d'une face de butée.

La face de butée portant l'élément conducteur peut présenter une ou plusieurs des caractéristiques optionnelles de la face de butée des moyens de butée décrits précédemment.

Dans un mode de réalisation, le support présente une surface concave, sensiblement sphérique et qui, en vue de face, présente une forme sensiblement rectangulaire, ladite surface étant conformée pour servir de moyens de butée et étant recouverte, au moins en partie, voire sensiblement totalement, par un revêtement conducteur électriquement pouvant servir d'électrode.

Le dispositif peut encore comporter des moyens de connexion électrique, et notamment des bornes de connexion électrique, permettant de connecter électriquement ladite ou lesdites électrodes aux bornes d'un générateur électrique.

Le dispositif peut comporter un réservoir de produit à injecter, notamment un réservoir solidaire du support, et des moyens pour transférer du produit depuis ce réservoir vers la ou les aiguilles d'injection.

Dans un deuxième mode de réalisation principal, l'invention concerne un dispositif d'injection destiné à l'injection d'un produit dans un oeil, le dispositif comportant un support et une aiguille d'injection, l'aiguille d'injection étant fixée sur ledit support ou le support étant apte à guider un coulissement de ladite aiguille, ledit support présentant une surface de contact concave, conformée pour ne pas s'étendre sur plus d'un quadrant d'une demi-sphère et adaptée pour épouser la forme de la surface de l'oeil, en particulier la surface sensiblement sphérique de la sclérotique. Cette surface de contact peut servir de face de butée et/ou de substrat pour une ou plusieurs électrodes, notamment non invasives, et/ou de substrat pour une marque de repérage.

Les autres caractéristiques, éventuellement optionnelles, du dispositif du premier mode de réalisation principal décrit précédemment, et en particulier les caractéristiques relatives au nombre d'aiguilles d'injection et d'électrodes, à leur forme et leur agencement, à la forme de la face de butée, ou de la marque de repérage, peuvent être appliquées, optionnellement, au dispositif du deuxième mode de réalisation principal.

Dans un troisième mode de réalisation principal, l'invention concerne un dispositif d'injection comportant :
- une aiguille d'injection orientée suivant une direction d'aiguille d'injection ;
- un support sur lequel ladite aiguille d'injection est fixée ou apte à guider un coulissement de l'aiguille d'injection,
- des moyens de butée, aptes à limiter la pénétration de l'aiguille d'injection suivant ladite direction d'aiguille d'injection, lesdits moyens de butée présentant une face de butée, de préférence de forme sensiblement sphérique,
le dispositif étant remarquable en ce que l'aiguille d'injection et l'axe principal de la face de butée s'étendent dans des plans parallèles, et en ce que, en projection dans l'un de ces plans, la direction de l'aiguille d'injection forme un angle δ supérieur à 20° et/ou inférieur à 85° avec l'axe principal de face de butée.

Les autres caractéristiques, éventuellement optionnelles, des dispositifs des premier et deuxième modes de réalisation décrits précédemment peuvent être appliquées, optionnellement, au dispositif selon le troisième mode de réalisation principal.

Dans un quatrième mode de réalisation principal, l'invention concerne un dispositif d'électroporation destiné à l'introduction d'un produit dans un oeil, le dispositif comportant :
- une électrode invasive,
- un support sur lequel l'électrode invasive est fixée ou apte à guider un coulissement de ladite électrode invasive,
le dispositif présentant une marque de repérage disposée de manière à pouvoir être mise en contact avec une région d'appui déterminée de la surface de l'oeil avant tout contact de l'électrode invasive avec ladite surface, puis être maintenue en contact avec ladite région d'appui pendant une pénétration normale de ladite électrode invasive à travers ladite surface de l'oeil.

L'électrode invasive peut en particulier être une aiguille d'injection.

Dans un cinquième mode de réalisation principal, l'invention concerne un dispositif d'électroporation destiné à l'introduction d'un produit dans un oeil, le dispositif comportant un support et une électrode invasive, l'électrode invasive étant fixée sur ledit support ou le support étant apte à guider un coulissement de ladite aiguille, ledit support présentant une surface de contact concave, conformée pour ne pas s'étendre sur plus d'un quadrant d'une demi-sphère et adaptée pour épouser la forme de la surface de l'oeil, en particulier la surface sensiblement sphérique de la sclérotique. Cette surface de contact peut servir de face de butée et/ou de substrat pour une ou plusieurs électrodes, notamment non invasives et/ou de substrat pour une marque de repérage.

Dans un sixième mode de réalisation principal, l'invention concerne un dispositif d'électroporation comportant :
- une électrode invasive orientée suivant une direction d'électrode invasive ;
- un support sur lequel ladite électrode invasive est fixée ou apte à guider un coulissement de l'électrode invasive ;
- des moyens de butée, de préférence solidaires du support, aptes à limiter la pénétration de l'électrode invasive suivant ladite direction d'électrode invasive, lesdits moyens de butée présentant une face de butée, de préférence de forme sensiblement sphérique,
le dispositif étant remarquable en ce que l'électrode invasive et l'axe principal de la face de butée s'étendent dans des plans parallèles, voire dans un même plan, et en ce que, en projection dans l'un de ces plans, la direction d'électrode invasive forme un angle supérieur à 20° et/ou inférieur à 85° avec l'axe principal de la face de butée.

Les caractéristiques, éventuellement optionnelles, des dispositifs des trois premiers modes de réalisation principaux décrits précédemment peuvent être appliquées, optionnellement, aux dispositifs des quatrième, cinquième et sixième modes de réalisation principaux.

En particulier, l'électrode invasive peut présenter une ou plusieurs des caractéristiques de l'aiguille d'injection des dispositifs selon les trois premiers modes de réalisation principaux de l'invention.

Le dispositif peut en particulier comporter trois électrodes invasives aptes à être connectées électriquement à une même borne d'un générateur électrique, ces trois électrodes invasives pouvant être conformées et agencées comme les aiguilles d'injection des trois premiers modes de réalisation principaux de l'invention.

L'invention concerne encore un dispositif d'électroporation comportant un dispositif d'injection selon l'invention comprenant au moins deux électrodes et un générateur électrique connecté électriquement aux dites électrodes pour pouvoir générer en champ électrique entre lesdites électrodes. De préférence, le générateur électrique est adapté pour favoriser l'électroportation d'un produit injecté dans un oeil au moyen dudit dispositif d'injection. De préférence, le dispositif d'injection comporte alors au moins une électrode invasive et une électrode non invasive.

Il est également décrit un procédé d'injection d'un produit dans un oeil au moyen d'un dispositif conforme au premier mode de réalisation principal de l'invention, ledit procédé ne formant pas partie de l'invention et comportant les étapes successives suivantes :
a) mise en contact de la marque de repérage avec une région d'appui de la surface de l'oeil ;
b) pénétration de la ou des aiguilles d'injection dans l'oeil en maintenant ladite marque de repérage en contact avec ladite région d'appui.

La région d'appui peut notamment être un rebord de la cornée à l'interface entre la cornée et la sclérotique.

Dans un exemple ne formant pas partie de l'invention, à l'étape b), on met en rotation la ou les aiguilles d'injection autour de la région d'appui.

Dans un exemple ne formant pas partie de l'invention, où la ou les aiguilles d'injection sont coulissantes par rapport au support, l'étape b) peut résulter d'un coulissement de ces aiguilles d'injection par rapport au support, le support ayant été immobilisé par appui sur la surface de l'oeil. En particulier, cette immobilisation peut résulter d'une complémentarité de forme entre la marque de repérage et le limbe de l'oeil.

Il est également décrit un procédé d'électroporation destiné à l'introduction d'un produit dans un oeil au moyen d'un dispositif conforme au quatrième mode de réalisation principal décrit ci-dessus, ledit procédé ne formant pas partie de l'invention et comportant les étapes successives suivantes :
a) mise en contact de la marque de repérage avec une région d'appui de la surface de l'oeil ;
b) pénétration de la ou des électrodes invasives dans l'oeil en maintenant ladite marque de repérage en contact avec ladite région d'appui ;
c) génération d'un champ électrique entre ladite électrode invasive et une autre électrode, par exemple une électrode non invasive également fixée sur le dispositif d'électroporation, le champ électrique étant adapté pour favoriser ladite électroporation.

La région d'appui peut notamment être un rebord de la cornée.

Dans un exemple ne formant pas partie de l'invention, à l'étape b), on met en rotation la ou les électrodes invasives autour de la région d'appui.

Dans un exemple ne formant pas partie de l'invention, où la ou les électrodes invasives sont coulissantes par rapport au support, l'étape b) peut résulter d'un coulissement de ces électrodes invasives par rapport au support, le support ayant été immobilisé par appui sur la surface de l'oeil. En particulier, cette immobilisation peut résulter d'une complémentarité de forme entre la marque de repérage et le limbe de l'oeil.

Dans un exemple ne formant pas partie de l'invention, la ou les électrodes invasives constituent des aiguilles d'injection et le procédé d'électroporation comporte une étape d'injection du produit. Cette étape d'injection peut avoir lieu avant, pendant ou après l'étape c).

### Brève description des figures

D'autres caractéristiques et avantages de l'invention apparaîtront encore à la lecture de la description détaillée qui va suivre et à l'examen du dessin annexé dans lequel :
- la figure 1 représente, en perspective, un premier mode de réalisation d'un dispositif d'injection selon l'invention destiné à une injection, avec électroporation, d'un produit dans un muscle ciliaire d'un oeil;
- la figure 2 représente une vue de dessus de ce dispositif ;
- les figures 3 et 4 représentent en coupe, suivant le plan transversal médian A-A et le plan transversal B-B représentés sur la figure 2, des sections du dispositif représenté sur la figure 1 ;
- la figure 5 représente, en perspective, un deuxième mode de réalisation d'un dispositif selon l'invention;
- la figure 6 représente la forme d'une face de butée d'un dispositif selon l'invention, dans un mode de réalisation préféré,
- la figure 7 représente très schématiquement un dispositif selon l'invention pendant une phase de pénétration. La figure 7 permet de visualiser l'angle β entre la direction de pénétration d'une aiguille d'injection au point de pénétration dans l'oeil et la direction de cette aiguille d'injection,
- la figure 8 représente en coupe, suivant un plan transversal médian, une section d'un dispositif selon l'invention selon un troisième mode de réalisation,
- la figure 9 représente la quantité de hTNFr-Is ayant pu pénétrer au sein de cellules selon que la dose injectée a été injectée en un point d'injection ou en plusieurs points d'injection,
- la figure 10 représente un détail de la figure 3, et
- la figure 11 représente une vue partielle du dispositif représenté sur la figure 10, observé selon l'axe Dcₒ par l'observateur Obs représenté sur la figure 10.

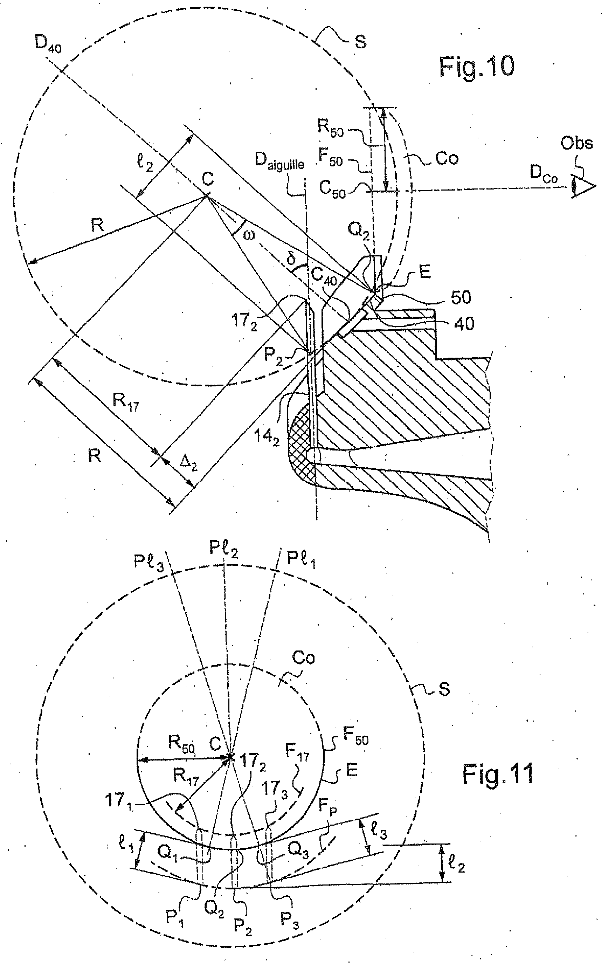

Sur les figures 10 et 11, le dispositif a été représenté dans une position de service, c'est-à-dire à la fin de la phase de pénétration, l'enveloppe sphérique S représentant schématiquement la sclérotique d'un oeil et Co représentant schématiquement la cornée de cet oeil.

Sur la figure 11, seules les parties des aiguilles à l'intérieur de l'enveloppe sphérique S sont représentées, en trait interrompu.

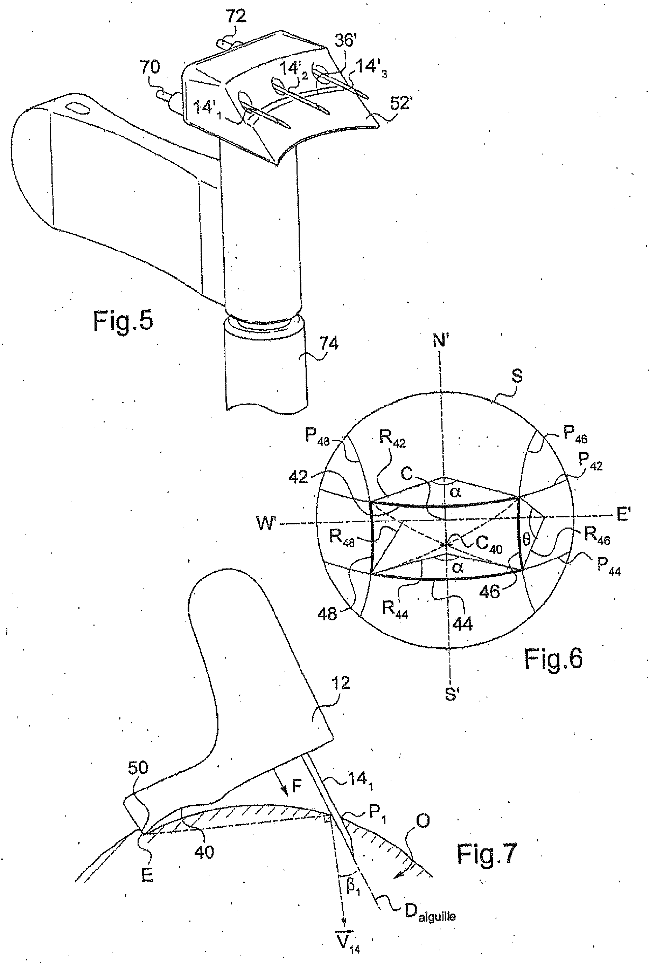

Dans les différentes figures, des références identiques sont utilisées pour désigner des organes identiques ou analogues. Sur la figure 5, les références sont affectées d'un signe '. Sur la figure 8, elles sont affectées d'un signe ".

### Définitions

La « phase de pénétration » d'une aiguille d'injection correspond à toute la phase pendant laquelle l'aiguille d'injection pénètre «normalement » dans l'oeil.

Par « pénétration normale » de l'aiguille d'injection, on entend une pénétration sensiblement selon la direction locale de l'aiguille d'injection au point de pénétration de cette aiguille d'injection dans l'oeil, c'est-à-dire au niveau du point de la surface de l'oeil par lequel l'aiguille d'injection traverse cette surface. Une telle pénétration correspond au mouvement pour lequel une aiguille d'injection est conçue, afin d'éviter que l'enfoncement de l'aiguille n'endommage inutilement la surface de l'oeil.

La marque de repérage et l'aiguille d'injection peuvent être configurées pour que, pendant toute la phase de pénétration de ladite aiguille d'injection dans l'oeil, la direction locale de l'aiguille d'injection au point de pénétration dans l'oeil forme, avec la direction de son mouvement d'avancement audit point de pénétration, ou « direction de pénétration », un angle β toujours inférieur à un angle d'écartement maximal βₘₐₓ de 15°, de préférence 10°, voire 5°.

La "direction locale de l'aiguille d'injection au point de pénétration dans l'oeil" est définie par la tangente à l'aiguille d'injection au point de l'oeil par lequel elle pénètre dans ce dernier. Dans le cas d'une aiguille rectiligne, cette direction est définie par l'axe de l'aiguille.

La "direction du mouvement d'avancement au point de pénétration" est la direction du vecteur vitesse de la région de l'aiguille située audit point de pénétration. Lorsque l'aiguille pénètre par une rotation du support autour d'un point d'appui, cette direction est donc définie par la tangente, au point de pénétration, au cercle centré sur ce point d'appui et passant par ledit point de pénétration.

Par exemple, avec une aiguille rectiligne, une pénétration « normale » correspond à un mouvement de l'aiguille selon l'axe de l'aiguille. La direction de pénétration forme alors un angle constant, pendant toute la phase de pénétration, avec la surface de l'oeil au point de pénétration de ladite aiguille d'injection. Cet angle peut être de 90°, mais une pénétration «normale» n'est pas limitée à cet angle.

De préférence, la phase de pénétration s'étend depuis le premier contact de l'aiguille d'injection avec la surface de l'oeil jusqu'à une position de service.

L'«axe principal» d'une surface est la direction perpendiculaire à une surface passant par son centre.

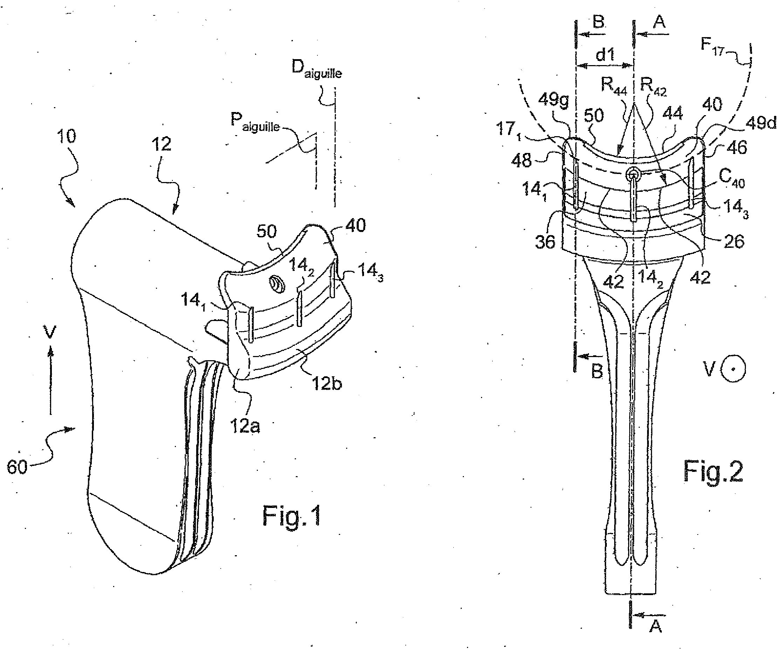

Dans la présente description, les adjectifs «supérieur» et «inférieur» sont définis par rapport à une direction verticale V telle que représentée sur les figures 1, 3 et 4.

Les adjectifs «droit» et «gauche» sont définis par rapport à la vue de la figure 2.

On appelle « quadrant d'une demi-sphère » un quart de la surface de cette demi-sphère obtenu par des coupes suivant deux plans perpendiculaires se coupant suivant l'axe principal de la demi-sphère.

Dans la présente description, par « comportant un » il y a lieu de comprendre sauf mention contraire, « comportant au moins un».

### Description détaillée d'un mode de réalisation

Le dispositif d'injection 10 représenté sur les figures 1 à 4 comporte un support 12 sur lequel sont fixées trois aiguilles d'injection 14₁, 14₂ et 14₃ identiques, rectilignes et orientées parallèlement les unes aux autres suivant une direction d'aiguille D_{aiguille}.

Les trois aiguilles d'injection appartiennent à un même plan, appelé «plan des aiguilles » et référencé P_{aiguilles}**.**

Afin d'être rendues solidaires du support 12, les aiguilles d'injection sont prises en sandwich entre une partie principale 12a du support 12 et une pièce de blocage 12b fixée sur la partie principale 12a, par exemple par clipsage, par collage ou par fusion de matière.

L'aiguille 14₂ s'étend à égale distance des aiguilles 14₁ et 14₃. La distance d₁ entre l'aiguille 14₂ et les aiguilles 14₁ et 14₃ peut être supérieure à 3 mm ou 3,5 mm ou 4 mm et inférieure à 6 mm ou 5,5 mm. En particulier, elle peut être d'environ 4,5 mm (figure 2).

Les trois aiguilles étant identiques et fixées de manière similaire sur le support, seule l'aiguille 14₁ est décrite en détail, au regard de la figure 4. Pour une même référence, les indices «1», «2» et «3» se rapportent à l'aiguille d'injection 14₁, 14₂ et 14₃, respectivement.

L'aiguille 14₁ s'étend depuis une extrémité proximale 16₁, noyée dans le support, jusqu'à une extrémité distale 17₁, en pointe biseautée afin de faciliter la pénétration de l'aiguille dans l'oeil, débouchant par un orifice d'éjection axial. Les extrémités distales 17₁, 17₂, et 17₃ des trois aiguilles 14₁, 14₂ et 14₃ s'étendant sensiblement suivant un arc de cercle F₁₇, comme représenté sur les figures 2 et 11. L'aiguille 14₁ est classiquement traversée par une lumière 18₁, destinée au transfert de produit depuis l'extrémité proximale jusqu'à l'extrémité distale. Le diamètre extérieur de l'aiguille 14₁ est par exemple d'environ 0,3 mm.

L'aiguille d'injection 14₁ comporte une partie en porte-à-faux 22₁, c'est-à-dire faisant saillie du support, et une partie insérée 24₁, noyée dans le support. La longueur l₂₂ de la partie en porte-à faux 22₁ peut être par exemple comprise entre 3,3 et 3,7 mm, une longueur de 3,5 mm étant bien adaptée. Les parties en porte-à-faux des trois aiguilles d'injection peuvent avoir une longueur sensiblement identique.

La partie en porte-à-faux 22₁ de l'aiguille 14₁ est recouverte sur une partie de sa longueur, d'un revêtement isolant 34₁. Le revêtement isolant 34₁ peut se prolonger sur la partie insérée de l'aiguille d'injection 14₁, voire recouvrir totalement cette partie insérée.

Les extrémités proximales des aiguilles débouchent dans un canal de distribution 20 commun en communication de fluide avec l'extérieur du support. Le canal de distribution 20 permet donc d'amener du produit depuis l'extérieur du support vers les extrémités proximales des aiguilles d'injection, puis, *via* les lumières de ces aiguilles d'injection, jusqu'aux extrémités distales des aiguilles d'injection. Le diamètre du canal de distribution 20, d₂₀, peut être supérieur à 0,5 mm ou 0,6 mm et/ou inférieur à 0,9 mm ou inférieur à 0,8 mm, un diamètre de 0,7 mm étant bien adapté.

Le dispositif comporte encore des moyens, non représentés, pour boucher une ou plusieurs des aiguilles, voire pour boucher l'entrée du canal de distribution 20.

Les aiguilles d'injection font saillie du support 12 à partir d'une face d'insertion 26 de forme sensiblement sphérique, c'est-à-dire portée par une enveloppe sphérique, afin de rester écartées de la surface de l'oeil dans la position de butée du support afin que l'utilisateur puisse observer le point de pénétration de l'aiguille.

La face d'insertion 26 est inclinée par rapport à la direction D_{aiguille}, puis rejoint une face intermédiaire 36 sensiblement plate, sensiblement parallèle au plan des aiguilles P_{aiguilles}, et écartée d'une distance d₃₆ de ce plan déterminée de manière que la distance entre la marque de repérage, sous la forme d'une bande 50 qui sera décrite ultérieurement, et les points de pénétration des différentes aiguilles dans l'enveloppe sphérique S, correspondant sensiblement à la surface de l'oeil dans la position de butée du support, soit d'environ 4 mm.

Du coté opposé à la face d'insertion 26, la face intermédiaire 36 se prolonge par une face de butée 40. La face de butée 40 s'étend suivant une enveloppe sphérique S de centre C et de rayon R, comme représenté sur les figures 4, 6 et 10. Le rayon R est d'environ 14,75 mm et correspond au rayon de la surface extérieure de l'oeil, dans la région de la sclérotique, à proximité de la cornée.

L'axe principal D₄₀ de la face de butée 40 est la droite coupant perpendiculairement la face de butée 40 en son centre C₄₀. L'axe principal passe donc par le centre C de l'enveloppe sphérique S. Il est inclus dans le plan médian A-A représenté sur la figure 2, (voir aussi la figure 10).

Dans le plan perpendiculaire au plan des aiguilles P_{aiguilles} et incluant l'aiguille 14₁, la face de butée 40 s'étend sur un secteur angulaire θ par exemple supérieur à 30° et inférieur à 40°, par exemple d'environ 36 degrés.

Dans le plan des aiguilles P_{aiguilles}, la face de butée 40 s'étend sur un secteur angulaire α par exemple supérieur à 80° et inférieur à 90°.

La face de butée 40 est délimitée par deux grands côtés, à savoir un bord supérieur 42 et un bord inférieur 44, sensiblement parallèles et circulaires, s'étendant dans des plans sensiblement parallèles au plan des aiguilles P_{aiguilles}, et par deux petits côtés, à savoir un bord latéral droit 46 et un bord latéral gauche 48, également sensiblement parallèles et circulaires, s'étendant dans des plans sensiblement parallèles au plan médian A-A.

Comme représenté sur la figure 6, sur laquelle l'enveloppe sphérique S est représentée, les bords supérieur 42 et inférieur 44 s'étendent ainsi selon des «parallèles» P₄₄ et P₄₂ de rayons R₄₄ et R₄₂, respectivement, les «parallèles» étant définis par rapport à un axe N'S', tandis que les bords latéraux droit et gauche 46 et 48, s'étendent suivant des parallèles P₄₆ et P₄₈, de rayons R₄₆ et R₄₈ respectivement, définis par rapport à un axe W'E' perpendiculaire à l'axe N'S' et coupant ce dernier au centre C.

Le rayon R₄₂ peut être supérieur à 8 mm, voire supérieur à 8,5 mm, voire 8,8 mm, ou 8,9 mm et/ou inférieur à 10 mm ou inférieur à 9,5 mm, ou 9,2 mm, voire 9,1 mm ou même 9 mm. Le rayon R₄₂ peut en particulier être de 8,94 mm.

Le rayon R₄₄ est défini de manière à ce que le bord inférieur 44 puisse épouser le rebord de la cornée.

Le rayon R₄₄ peut être supérieur à 6 mm ou 6,4 mm ou même 6,5 mm et/ou inférieur à 7 mm, ou 6,8 mm, ou même 6, 7mm. Un rayon R₄₄ de 6,58 mm est bien adapté.

Vue de face, la face de butée 40 présente donc une forme sensiblement rectangulaire dont le centre C₄₀ est à mi-distance entre le bord inférieur 44 et le bord supérieur 42, et entre le bord latéral droit 46 et le bord latéral gauche 48.

Les angles 49_{d} et 49_{g} entre le bord inférieur 44 et les bords latéraux droit et gauche 46 et 48, respectivement, sont de préférence arrondis afin de limiter les risques de blessure. Le rayon de courbure des arrondis peut par exemple être de 1 mm.

La distance entre les deux bords latéraux droit et gauche 46 et 48, mesurée selon la direction W'E' peut être supérieure à 17 mm, 19 mm ou 20 mm et/ou inférieure à 25 mm, 23 mm ou 22 mm. Elle peut être par exemple de 21,11 mm.

La distance entre les deux bords supérieur 42 et inférieur 44, mesurée selon la direction N'S' peut être supérieure à 1,9 mm, 2,2 mm, voire 2,3 mm et/ou inférieure à 3 mm, 2,6 mm, voire 2,5 mm. Une hauteur de 2,4 mm est bien adaptée.

Dans le mode de réalisation représenté, le bord inférieur 44 est délimité par une bande 50 en un matériau souple, par exemple en silicone. Le reste de la face de butée 40 est couvert par un revêtement 52 conducteur électriquement.

Comme on le verra dans la suite de la description, la bande 50 est une marque de repérage et le revêtement 52 peut servir d'électrode non invasive.

Dans le plan de coupe de la figure 4, la distance D₅₀ entre la bande 50 et le point de pénétration P₁ de l'aiguille d'injection 14₁ dans l'enveloppe sphérique S, est de préférence supérieure à 1 mm, supérieure à 2 mm, supérieure à 3 mm, supérieure à 3,5 mm et/ou inférieure à 6 mm, inférieure à 5 mm, voire inférieure à 4, 5 mm. De préférence, cette caractéristique est respectée pour chacune des aiguilles, en considérant chaque fois un plan de coupe contenant l'aiguille considérée et parallèle au plan B-B. Une distance de 4 mm est bien adaptée.

Comme représenté sur la figure 11, la bande 50 matérialise une ligne de repérage qui s'étend selon un arc de cercle F₅₀ dont le rayon R₅₀ est sensiblement égal au rayon du rebord E de la cornée Co, c'est-à-dire compris entre 5 et 8 mm, de préférence compris entre 6 et 7 mm. Le centre de l'arc de cercle F₅₀ est noté C₅₀. Son axe est noté « D_{Co} » et passe sensiblement par le centre C de l'enveloppe sphérique sur laquelle s'étend la face de butée 40.

Dans la position de service, la face de butée est donc complètement en appui sur la sclérotique de l'oeil et la marque de repérage est complètement en appui sur le rebord de la cornée.

« i » désignant l'indice de l'aiguille 14ᵢ, on désigne par :
- Pᵢ le point de pénétration de l'aiguille 14ᵢ dans l'enveloppe sphérique S, correspondant virtuellement à la sclérotique de l'oeil ;
- Plᵢ le plan passant par le centre C de l'enveloppe sphérique S, par le point de pénétration Pᵢ et qui coupe perpendiculairement l'arc de cercle F₅₀(le plan de coupe médian A-A correspondant ainsi au plan Pl₂) ;
- Qᵢ le point de l'arc de cercle F₅₀ par lequel le plan Plᵢ coupe l'arc de cercle F₅₀ ;
- lᵢ la distance entre les points Qᵢ et Pᵢ ;
- Δᵢ la distance minimale entre la surface de l'enveloppe sphérique S et l'extrémité distale 17ᵢ de l'aiguille 14ᵢ, ou « profondeur Δᵢ » ;
- ωᵢ l'angle, ayant pour sommet le centre C, entre le point Qᵢ et le point de pénétration Pᵢ.

Dans les plans Pl₁, Pl₂ et Pl₃, l'enveloppe sphérique S est donc coupée suivant un grand cercle et la cornée Co, coupée par son centre, présente une section d'aire sensiblement maximale.

Tous les points de pénétration Pᵢ des aiguilles 14ᵢ, appartiennent à un même cercle F_{P}. Les extrémités distales 17₁, 17₂ et 17₃ desdites aiguilles appartiennent à un même cercle F₁₇. Les cercles F₅₀, F_{P} et F₁₇ sont coaxiaux d'axe Dcₒ. Quelle que soit l'aiguille considérée, la distance entre le centre C et l'extrémité distale de ladite aiguille, R₁₇, est sensiblement constante. Il en est de même de la profondeur Δᵢ desdites extrémités distales par rapport à la surface de l'enveloppe sphérique S. Enfin, quelle que soit l'aiguille considérée, l'angle ω est sensiblement le même.

Quelle que soit l'aiguille 14ᵢ considérée, la distance lᵢ est supérieure à 1 mm, de préférence supérieure à 1,5 mm, de préférence encore supérieure à 1,8 mm et inférieure à 3 mm, de préférence inférieure à 1,5 mm, de préférence encore inférieure à 1,2 mm, et la profondeur Δᵢ est supérieure à 0,5 mm, de préférence supérieure à 0,6 mm, de préférence encore supérieure à 0,8 mm et inférieure à 1,5 mm, de préférence inférieure à 1,3 mm, de préférence encore inférieure à 1,2 mm. Ce mode de réalisation est particulièrement bien adapté pour une injection dans le muscle ciliaire de l'oeil.

Dans un autre mode de réalisation non représenté, quelle que soit l'aiguille 14ᵢ considérée, la distance lᵢ est supérieure à 2 mm, de préférence supérieure à 3 mm et inférieure à 4,5 mm, de préférence inférieure à 4 mm, voire inférieure à 3,5 mm, et la distance Δᵢ est supérieure à 6 mm, de préférence supérieure à 8 mm et inférieure à 15 mm, de préférence inférieure à 13 mm. Ce mode de réalisation est particulièrement bien adapté pour une injection dans le corps vitréen.

L'angle δ entre l'axe principal D₄₀ de la face de butée 40 et la direction d'aiguille D_{aiguille} de l'aiguille 14₂ est compris entre 50 et 60 degrés, par exemple d'environ 55 degrés (figure 10). Avantageusement, de tels angles δ permettent un positionnement de la marque de repérage 50 en appui sur le rebord de la cornée adapté pour une injection dans le muscle ciliaire.

L'angle δ, la distance d₃₆ et la longueur des aiguilles sont déterminées de manière que les aiguilles puissent pénétrer dans l'oeil jusqu'à une profondeur supérieure à 700 µm et inférieure à 3mm dans la position de butée du support. Avantageusement, cette profondeur permet une injection dans le muscle ciliaire particulièrement performante.

Le dispositif représenté comporte encore une poignée 60 facilitant sa manipulation. La longueur hors tout «L» du dispositif est par exemple d'environ 35 mm et sa largeur hors tout «l» est par exemple d'environ 21 mm (figure 3).

Dans le mode de réalisation représenté, le dispositif comporte encore des moyens pour connecter électriquement une ou plusieurs des aiguilles d'injection aux bornes d'un générateur d'un signal électrique. Par exemple les deux aiguilles d'injection 14₁ et 14₃ peuvent être connectées électriquement à une première des bornes de ce générateur tandis que l'aiguille d'injection centrale 14₂ peut être connectée à l'autre borne de ce générateur.

Le dispositif peut également comporter des moyens pour connecter électriquement le revêtement 52 à une des bornes du générateur.

Comme représenté sur la figure 4, le support peut en particulier comporter des conduits 62 permettant l'introduction de broches de connexion électrique avec une ou plusieurs des aiguilles d'injection, voire avec toutes les aiguilles d'injection. La transmission du courant électrique peut se faire, de préférence, par l'intermédiaire du canal de distribution 20, par exemple si ce canal comporte une paroi 64 en un matériau conducteur électriquement.

Dans le mode de réalisation préféré de l'invention, les trois aiguilles 14₁, 14₂ et 14₃ sont connectées électriquement à une première borne du générateur tandis que le revêtement 52 est connecté à la deuxième borne de ce générateur.

La figure 5 représente une variante du dispositif décrit au regard des figures 1 à 4. Dans cette variante, la largeur de la face intermédiaire 36 est réduite afin de minimiser l'encombrement, et ainsi faciliter le positionnement du dispositif entre les deux paupières d'un sujet vigile. La figure 5 permet également de visualiser les broches 70 et 72 permettant de connecter électriquement aux bornes d'un générateur un premier ensemble d'électrodes constitué par un revêtement 52' et un deuxième ensemble d'électrodes constitué par des aiguilles d'injection 14₁', 14₂' et 14₃'.

Le dispositif représenté sur la figure 5 comporte également un réservoir 74 de produit en communication de fluide avec un canal de distribution, non représenté, commun aux trois aiguilles d'injection. Des moyens pour transférer du produit, non représentés, par exemple un piston apte à expulser le produit hors du réservoir vers le canal de distribution, sont également prévus.

La figure 8 représente une variante d'un dispositif selon l'invention notamment destiné à une injection dans la cavité vitréenne d'un oeil.

Le dispositif représenté, en coupe médiane, sur la figure 8, comporte un support 12" et une unique aiguille d'injection 14".

Le support 12"comporte des moyens de butée du support, en l'occurrence une face de butée 40" qui s'étend suivant une enveloppe sphérique S. L'enveloppe sphérique S correspond sensiblement à la surface de l'oeil dans la position de butée du support où la face de butée 40" est en contact avec la surface de l'oeil. Le centre C de cette enveloppe sphérique correspond ainsi sensiblement au centre du globe oculaire.

La longueur de l'aiguille d'injection introduite dans l'oeil est beaucoup plus grande que celle du dispositif décrit précédemment, destiné à une injection dans le muscle ciliaire. De préférence, cette longueur est comprise entre 10 et 13 mm, et par exemple d'environ 12 mm. Une telle longueur de l'aiguille d'injection ne pourrait pénétrer, à moins que l'aiguille d'injection ne soit courbe, par rotation autour de la bande 50, sans endommager la surface de l'oeil si l'aiguille d'injection 14" était fixée sur le support.

C'est pourquoi l'aiguille d'injection 14" n'est pas fixée sur le support mais peut coulisser dans un orifice traversant 82 du support entre une position rétractée, non représentée, et une position de butée d'aiguille, ou « position sortie », telle que représentée sur la figure 8.

Pour déterminer la position de butée d'aiguille, l'aiguille d'injection 14" comporte des moyens de butée d'aiguille 80 aptes à limiter la longueur de la partie en porte-à-faux 22" de l'aiguille d'injection 14" pouvant faire saillie du support 12".

La distance D₅₀" entre la marque de repérage 50" et le point de pénétration P" de l'aiguille d'injection 14" dans l'enveloppe sphérique S est par exemple d'environ 4 mm.

Dans le mode de réalisation représenté, l'aiguille d'injection 14" présente une longueur telle que, dans la position de butée d'aiguille, son extrémité distale soit à proximité du centre C.

Dans un mode de réalisation, le dispositif comporte encore des moyens permettant de projeter l'aiguille depuis sa position rétractée jusqu'à sa position sortie. Avantageusement, la position sortie peut ainsi être obtenue de manière rapide et efficace, par exemple par simple manipulation d'un bouton d'actionnement.

Le mode de réalisation de la figure 8 est particulièrement avantageux pour des aiguilles devant pénétrer dans l'oeil sur une longueur supérieure à 5 mm, supérieure à 7 mm, voire supérieure à 10 mm et/ou inférieure à 15 mm.

Pour utiliser un dispositif tel que ceux décrits sur les figures 1 à 6, l'opérateur peut procéder suivant les étapes suivantes :
Dans un premier temps, l'opérateur accouple le dispositif avec une source de produit, par exemple une seringue remplie de produit. Il connecte ensuite électriquement les premier et deuxième ensembles d'électrodes aux deux bornes d'un générateur électrique. Le dispositif est alors prêt à être appliqué sur l'oeil dans lequel une injection de produit est souhaitée.

Pour positionner le dispositif, comme représenté sur la figure 7, l'opérateur appuie la marque de repérage 50 sur le rebord E de la cornée de l'oeil O, en prenant soin que les pointes des aiguilles d'injection ne viennent pas en contact avec l'oeil. L'appui sur le rebord de la cornée permet avantageusement un positionnement stable, mais également très précis.

Tout en maintenant la marque de repérage 50 en contact avec le rebord de la cornée, l'opérateur fait alors pénétrer les aiguilles d'injection à travers la surface de l'oeil par rotation du support 12 autour de la marque de repérage 50 (flèche F). Les trois aiguilles pénètrent sensiblement simultanément à travers cette surface.

Comme représenté sur la figure 7, au point de pénétration P₁ de l'aiguille 14₁ dans l'oeil O, la direction de pénétration *̅V̅*̅₁₄ de l'aiguille 14₁ forme un angle β₁ avec la direction d'aiguille D_{aiguille}. L'agencement et la forme des aiguilles 14₁, 14₂, et 14₃ permettent à leurs directions de pénétration respectives de former, à leurs points de pénétration respectifs, des angles β₁, β₂ et β₃ avec la direction d'aiguille D_{aiguille} sensiblement égaux à β. De préférence, la longueur des aiguilles d'injection et/ou leur forme sont déterminées de manière que, pendant toute la phase de pénétration, en l'occurence jusqu'à la position de butée du support, cet angle reste inférieur à 15°, de préférence 10°, voire 5°, quel que soit le degré d'enfoncement atteint.

La forme de la face de butée, et notamment le fait qu'elle ne s'étend pas sur plus d'un quadrant d'une demi-sphère, facilite la mise en position du dispositif et sa manipulation pendant la phase de pénétration des aiguilles d'injection.

L'opérateur poursuit le mouvement de pénétration jusqu'à ce que la face de butée 40 vienne, dans une position de butée du support, épouser la sclérotique.

Les inventeurs ont montré que les yeux des êtres humains présentent tous des dimensions et des formes très proches et, en particulier, que la distance entre le muscle ciliaire et le rebord de la cornée d'un oeil est sensiblement la même quel que soit l'individu considéré. La forme et l'agencement des aiguilles d'injection, de la marque de repérage et de la face de butée sont déterminés pour garantir à l'opérateur que, dans la position de butée du support, les orifices d'éjection des aiguilles d'injection débouchent dans le muscle ciliaire. Dans la position de butée du support, l'opérateur sait donc que les aiguilles d'injection sont parfaitement positionnées et que le produit sera bien injecté dans le muscle ciliaire. Il immobilise alors le dispositif dans cette position. L'appui de la face de butée 40 sur la sclérotique et de la marque de repérage 50 sur le rebord de la cornée garantissent une bonne stabilité du dispositif.

L'agencement des électrodes invasives, en l'occurrence des aiguilles d'injection 14₁, 14₂ et 14₃, par rapport à l'électrode non invasive, en l'occurrence le revêtement 52, permet de créer un champ électrique particulièrement efficace pour l'électroporation.

L'opérateur peut alors commencer l'injection du produit en agissant sur le piston de la seringue.

L'opérateur envoie alors un signal électrique adapté, par exemple des impulsions électriques adaptées, grâce au générateur électrique, de manière à créer, dans la zone d'injection, un champ électrique favorisant l'électroporation.

L'augmentation de la pression locale au niveau des points d'injection est réputée pour favoriser l'introduction du produit injecté dans les cellules, et notamment dans le cas d'une transfection. L'homme du métier considère donc généralement qu'il est préférable de limiter le nombre de points d'injection, si possible en utilisant qu'une seule aiguille d'injection. De manière surprenante, les inventeurs ont cependant constaté que la multiplication des points d'injection favorise la pénétration du produit, comme cela peut être déduit de la figure 9. Un dispositif à trois aiguilles s'est avéré particulièrement efficace.

La figure 9 montre en effet que le résultat d'une injection d'un volume de 10 µl contenant 30 µg de plasmide codant pour un récepteur soluble au TNF est inférieur à celui d'une injection de 30 µl contenant également 30µg dudit plasmide (effet hydrodynamique), lui-même inférieur à l'injection de trois volumes de 10µl contenant chacun 10 µg du plasmide simultanément par trois aiguilles d'injection.

Ceci démontre que la multi injection, c'est-à-dire simultanément par plusieurs points d'injection, est préférable à une unique injection à dose égale de plasmide.

L'injection du produit terminée, l'opérateur déconnecte électriquement les électrodes et le générateur, puis extrait les aiguilles d'injection de l'oeil.

La mise en oeuvre du dispositif représenté sur la figure 8 diffère de celui décrit pour le dispositif représenté sur les figures 1 à 6 en ce que le positionnement du support peut être effectué indépendamment de la pénétration de l'aiguille d'injection 14" dans l'oeil.

Dans un premier temps, comme décrit précédemment, l'opérateur appuie la marque de repérage 50 sur le rebord de la cornée de l'oeil, puis vient appuyer la face de butée 40" sur cette surface, l'aiguille d'injection 14" étant en position rétractée, par exemple complètement extraite de l'orifice traversant 82.

Le support étant dans la position de butée du support, l'opérateur peut alors sortir l'aiguille d'injection du support et en faire pénétrer l'extrémité distale dans l'oeil, puis pousser cette aiguille d'injection jusqu'à la position de butée d'aiguille représentée sur la figure 8, où les moyens de butée d'aiguille 80 prennent appui sur le support 12".

Ce mode de réalisation de l'invention présente l'avantage d'empêcher tout contact involontaire de l'aiguille d'injection avec la surface de l'oeil. En outre, il permet de faire pénétrer une grande longueur d'aiguille dans l'oeil.

Les autres étapes d'injection et/ou de génération d'un signal électrique favorisant l'électroporation sont identiques à celles décrites précédemment.

Le découplage du dispositif représenté sur la figure 8 est également particulièrement avantageux. En effet, l'opérateur peut extraire l'aiguille d'injection 14" de l'oeil en bénéficiant du guidage résultant du coulissement de l'aiguille d'injection 14" dans l'orifice traversant 82, agissant comme un rail de coulissement pour l'aiguille d'injection 14". Le risque d'endommagement de l'oeil est alors particulièrement réduit.

Comme cela apparaît clairement à présent, le dispositif selon l'invention permet un positionnement très précis à la surface de l'oeil, avant toute pénétration d'une aiguille d'injection et/ou d'une électrode invasive. Il permet également de guider ces aiguille(s) et électrode(s) pendant la phase de pénétration.

Bien entendu, l'invention, définie par les revendications attachées, n'est pas limitée aux modes de réalisation décrits et représentés, fournis à titre illustratif.

En particulier, les aiguilles d'injection pourraient ne pas constituer des électrodes invasives. La face de butée pourrait également ne pas porter une électrode non invasive.

Réciproquement, dans d'autres modes de réalisation, les aiguilles pourraient ne pas être des aiguilles d'injection, mais seulement servir d'électrodes invasives.

En particulier, les différents modes de réalisation pourraient être combinés. Il serait notamment possible d'utiliser des aiguilles rétractables ou, de manière plus générale, des aiguilles pouvant coulisser sur le support, avec des dispositifs destinés au traitement d'un muscle ciliaire, c'est-à-dire avec des aiguilles relativement petites.

Enfin, la forme du dispositif n'est pas limitée à celles représentée. Le dispositif pourrait en et à titre d'exemple ne formant pas partie de l'invention être adapté pour l'injection de produit ou l'électroporation dans d'autres muscles que le muscle ciliaire, un tel dispositif ne formant toutefois pas partie de l'invention qui est définie par les revendications.

## Revendications

1. Dispositif d'injection destiné à l'injection d'un produit dans un oeil, ou d'électroporation, comportant :
- une aiguille sous la forme d'une aiguille d'injection et/ou d'une électrode invasive (14₁, 14₂, 14₃; 14₁', 14₂', 14₃'),
- un support (12) sur lequel l'aiguille est fixée ou apte à guider un coulissement de l'aiguille suivant l'axe de ladite aiguille,
le dispositif étant **caractérisé en ce qu'**il comporte une marque de repérage (50) fixée sur le support et disposée suivant un arc de cercle d'un rayon supérieur à 5 mm et inférieur à 8 mm, la longueur dudit arc de cercle étant inférieure à 20 mm, de manière à pouvoir être mise en contact avec une région d'appui (E) déterminée de la surface de l'oeil (O) avant tout contact de l'aiguille (14₁, 14₂, 14₃; 14₁', 14₂', 14₃') avec ladite surface, puis être maintenue en contact avec ladite région d'appui pendant une phase de pénétration de ladite aiguille à travers ladite surface de l'oeil, la région d'appui étant au moins une partie du rebord (E) de la cornée de l'oeil (O), ladite pénétration résultant d'une rotation du support (12) autour de la marque de repérage (50) lorsque ladite aiguille est fixée sur le support (12).

2. Dispositif selon la revendication précédente, dans lequel l'aiguille (14₁, 14₂,14₃ ; 14₁', 14₂', 14₃') est conformée de manière que, pendant toute la phase de pénétration de ladite aiguille dans l'oeil, la direction locale (D_{aiguille}) de l'aiguille au point de pénétration (P₁) dans l'oeil (O) forme, avec la direction de pénétration (*̅V̅*̅₁₄) audit point de pénétration (P₁), un angle β (β₁, β₂, β₃) toujours inférieur à un angle d'écartement maximal βₘₐₓ de 15°.

3. Dispositif selon l'une quelconque des revendications précédentes, dans lequel l'aiguille est conformée de manière que son extrémité distale (17₁), puisse atteindre le muscle ciliaire de l'oeil (O) lors de ladite phase de pénétration de l'aiguille.

4. Dispositif selon l'une quelconque des revendications précédentes, dans lequel l'aiguille est, au moins en partie, en un matériau conducteur électriquement, le dispositif comportant des moyens de connexion permettant une connexion électrique de ladite aiguille à un générateur électrique.

5. Dispositif selon l'une quelconque des revendications précédentes, comportant des moyens de butée du support aptes à limiter le mouvement du support lors de la phase de pénétration, lesdits moyens de butée étant conformés de manière à limiter la longueur de ladite aiguille pouvant être enfoncée dans l'oeil, lors de ladite phase de pénétration, à moins de 20 mm.

6. Dispositif selon la revendication précédente, dans lequel les moyens de butée sont conformés de manière à limiter la longueur de ladite aiguille pouvant être enfoncée dans l'oeil, lors de ladite phase de pénétration, à moins de 3 mm.

7. Dispositif selon l'une quelconque des deux revendications immédiatement précédentes, dans lequel les moyens de butée comportent une face de butée (40) s'étendant sensiblement suivant une enveloppe sphérique, le rayon de courbure de ladite enveloppe sphérique étant supérieur à 9 mm et inférieur à 14 mm.

8. Dispositif selon la revendication 7, dans lequel la face de butée (40) ne s'étend pas sur plus d'un quadrant d'une demi-sphère.

9. Dispositif selon l'une quelconque des revendications 7 à 8, comportant plusieurs aiguilles s'étendant à l'intérieur de ladite enveloppe sphérique jusqu'à atteindre une profondeur identique.

10. Dispositif de l'une quelconque des revendications 5 à 9, dans lequel lesdits moyens de butée comportent une face de butée s'étendant suivant une enveloppe sphérique (S) et dans lequel la marque de repérage est une ligne de repérage en arc de cercle (F₅₀), l'axe dudit axe de cercle (Dcₒ) passant par le centre (C) de ladite enveloppe sphérique (S).

11. Dispositif selon l'une quelconque des revendications précédentes, dans lequel le point d'insertion de ladite aiguille dans ledit support est à l'extérieur de la surface sphérique virtuelle dont un grand cercle inclut la marque de repérage.

12. Dispositif de l'une quelconque des revendications 5 à 10, dans lequel lesdits moyens de butée comportent une face de butée s'étendant sensiblement suivant une enveloppe sphérique (S), la marque de repérage s'étend selon une ligne de repérage en arc de cercle (F₅₀), au moins une aiguille pénètre dans l'enveloppe sphérique par un point de pénétration (Pᵢ) et, dans un plan (Plᵢ) passant par le centre (C) de ladite enveloppe sphérique (S), par ledit point de pénétration (Pᵢ) et coupant perpendiculairement ledit arc de cercle (F₅₀), la distance (lᵢ) entre ledit point de pénétration (Pᵢ) et le point (Qᵢ) dudit arc de cercle par lequel ledit plan (Plᵢ) coupe ledit arc de cercle (F₅₀) est
(1) supérieure à 1 mm et inférieure à 3 mm, ladite aiguille s'étendant à l'intérieur de ladite enveloppe sphérique jusqu'à une profondeur (Δᵢ) supérieure à 0,5 mm et inférieure à 1,5 mm ; ou
(2) supérieure à 2 mm et inférieure à 4,5 mm, ladite aiguille s'étendant à l'intérieur de ladite enveloppe sphérique jusqu'à une profondeur (Δᵢ) supérieure à 6 mm et inférieure à 15 mm.

13. Dispositif selon l'une quelconque des revendications précédentes, comportant un jeu d'électrodes constitué de premier (14₁, 14₂, 14₃ ; 14₁', 14₂', 14₃') et deuxième (52) ensembles d'électrodes destinés à être connectés électriquement à des première et deuxième bornes, respectivement, d'un générateur électrique, et dans lequel ladite ou lesdites aiguilles constituent des électrodes invasives dudit premier ensemble d'électrodes.

14. Dispositif selon la revendication précédente, dans lequel le deuxième ensemble d'électrode comporte un revêtement conducteur électriquement, et dans lequel ledit revêtement (52) recouvre, au moins partiellement, une face de butée (40).

15. Dispositif selon l'une quelconque des revendications précédentes, dans lequel la marque de repérage est conformée pour prendre appui sur plus de 5 mm du rebord de la cornée.

16. Dispositif selon l'une quelconque des revendications précédentes, comportant au moins trois aiguilles, les extrémités distales desdites aiguilles formant un arc de cercle, celui-ci n'étant pas perpendiculaire à la direction locale desdites aiguilles au niveau de leur extrémité distale, la position desdites aiguilles considérée étant celle qu'elles occupent dans la position de butée lorsqu'elles sont montées coulissantes sur ledit support.

## Patentansprüche

1. Injektionsvorrichtung, die für die Injektion eines Produkts in ein Auge bestimmt ist, oder zur Elektroporation, umfassend:
- eine Nadel in Form einer Injektionsnadel und/oder einer invasiven Elektrode (14₁, 14₂, 14₃; 14₁', 14₂', 14₃'),
- einen Träger (12), auf dem die Nadel befestigt ist, oder der geeignet ist, ein Gleiten der Nadel entlang der Achse der Nadel zu führen,
wobei die Vorrichtung **dadurch gekennzeichnet ist, dass** sie eine Bezugsmarke (50) umfasst, die auf dem Träger befestigt und kreisbogenförmig mit einem Radius über 5 mm und unter 8 mm angeordnet ist, wobei die Länge des Kreisbogens kleiner als 20 mm ist, um mit einer bestimmten Auflageregion (E) der Oberfläche des Auges (0) vor jedem Kontakt der Nadel (14₁, 14₂, 14₃; 14₁', 14₂', 14₃') mit der Oberfläche in Kontakt gebracht zu werden, dann mit der Auflageregion während einer Eindringphase der Nadel durch die Oberfläche des Auges in kontakt gehalten zu werden, wobei die Auflageregion zumindest teilweise ein Rand (E) der Hornhaut des Auges (0) ist, wobei sich das Eindringen aus einer Rotation des Trägers (12) um die Bezugsmarke (50) ergibt, wenn die Nadel auf dem Träger (12) befestigt ist.

2. Vorrichtung nach dem vorhergehenden Anspruch, bei der die Nadel (14₁, 14₂, 14₃; 14₁', 14₂', 14₃') derart ausgebildet ist, dass während der gesamten Eindringphase der Nadel in das Auge die lokale Richtung (D_{Nadel}) der Nadel am Eindringpunkt (P₁) in das Auge (O) mit der Eindringrichtung (*̅V̅*̅₁₄) am Eindringpunkt (P₁) einen Winkel β (β₁, β₂, β₃) bildet, der immer kleiner als ein maximaler Abstandswinkel βₘₐₓ von 15° ist.

3. Vorrichtung nach einem der vorhergehenden Ansprüche, bei der die Nadel derart ausgebildet ist, dass ihr distales Ende (17₁) den Ziliarmuskel des Auges (O) während der Eindringphase der Nadel erreichen kann.

4. Vorrichtung nach einem der vorhergehenden Ansprüche, bei der die Nadel zumindest teilweise aus einem elektrisch leitfähigen Material ist, wobei die Vorrichtung Anschlussmittel umfasst, die einen elektrischen Anschluss der Nadel an einen elektrischen Generator ermöglichen.

5. Vorrichtung nach einem der vorhergehenden Ansprüche, umfassend Anschlagmittel des Trägers, die geeignet sind, die Bewegung des Trägers während der Eindringphase zu begrenzen, wobei die Anschlagmittel derart ausgebildet sind, dass sie die Länge der Nadel, die in das Auge eingeführt werden kann, während der Eindringphase auf weniger als 20 mm begrenzen.

6. Vorrichtung nach dem vorhergehenden Anspruch, bei der die Anschlagmittel derart ausgebildet sind, dass sie die Länge der Nadel, die in das Auge eingeführt werden kann, während der Eindringphase auf weniger als 3 mm begrenzen.

7. Vorrichtung nach einem der zwei unmittelbar vorhergehenden Ansprüche, bei der die Anschlagmittel eine Anschlagfläche (40) umfassen, die sich im Wesentlichen entlang einer kugelförmigen Umhüllenden erstreckt, wobei der Krümmungsradius der kugelförmigen Umhüllenden größer als 9 mm und kleiner als 14 mm ist.

8. Vorrichtung nach Anspruch 7, bei der sich die Anschlagfläche (40) nicht mehr als über einen Quadranten einer Halbkugel erstreckt.

9. Vorrichtung nach einem der Ansprüche 7 bis 8, umfassend mehrere Nadeln, die sich innerhalb der kugelförmigen Umhüllenden erstrecken, bis sie eine identische Tiefe erreichen.

10. Vorrichtung nach einem der Ansprüche 5 bis 9, bei der die Anschlagmittel eine Anschlagfläche umfassen, die sich entlang einer kugelförmigen Umhüllenden (S) erstreckt, und bei der die Bezugsmarke eine kreisbogenförmige Bezugslinie (F₅₀) ist, wobei die Achse der Kreisachse (Dcₒ) durch den Mittelpunkt (C) der kugelförmigen Umhüllenden (S) verläuft.

11. Vorrichtung nach einem der vorhergehenden Ansprüche, bei der der Einsetzpunkt der Nadel in den Träger außerhalb der virtuellen Kugelfläche, von der ein großer Kreis die Bezugsmarke einschließt, angeordnet ist.

12. Vorrichtung nach einem der Ansprüche 5 bis 10, bei der die Anschlagmittel eine Anschlagfläche umfassen, die sich im Wesentlichen entlang einer kugelförmigen Umhüllenden (S) erstreckt, wobei sich die Bezugsmarke entlang einer kreisbogenförmigen Bezugslinie (F₅₀) erstreckt, wobei mindestens eine Nadel in die kugelförmige Umhüllende durch einen Eindringpunkt (Pᵢ) eindringt, und wobei in einer Ebene (Plᵢ), die durch den Mittelpunkt (C) der kugelförmigen Umhüllenden (S), durch den Eindringpunkt (Pᵢ) verläuft und senkrecht den Kreisbogen (F₅₀) schneidet, der Abstand (lᵢ) zwischen dem Eindringpunkt (Pᵢ) und dem Punkt (Qᵢ) des Kreisbogens, durch den die Ebene (Plᵢ) den Kreisbogen (F₅₀) schneidet:
(1) größer als 1 mm und kleiner als 3 mm ist, wobei sich die Nadel innerhalb der kugelförmigen Umhüllenden bis zu einer Tiefe (Δᵢ) größer als 0,5 mm und kleiner als 1,5 mm erstreckt; oder
(2) größer als 2 mm und kleiner als 4,5 mm ist, wobei sich die Nadel innerhalb der kugelförmigen Umhüllenden bis zu einer Tiefe (Δᵢ) größer als 6 mm und kleiner als 15 mm erstreckt.

13. Vorrichtung nach einem der vorhergehenden Ansprüche, umfassend einen Elektrodensatz, der von ersten (14₁, 14₂, 14₃; 14₁', 14₂', 14₃') und zweiten (52) Elektrodeneinheiten gebildet ist, die dazu bestimmt sind, elektrisch an erste bzw. zweite Klemmen eines elektrischen Generators angeschlossen zu sein, und bei der die Nadel (n) invasive Elektroden der ersten Elektrodeneinheit darstellen.

14. Vorrichtung nach dem vorhergehenden Anspruch, bei der die zweite Elektrodeneinheit eine elektrisch leitfähige Verkleidung umfasst, und bei der die Verkleidung (52) zumindest teilweise eine Anschlagfläche (40) bedeckt.

15. Vorrichtung nach einem der vorhergehenden Ansprüche, bei der die Bezugsmarke ausgebildet ist, um auf mehr als 5 mm des Randes der Hornhaut zur Auflage zu gelangen.

16. Vorrichtung nach einem der vorhergehenden Ansprüche, mindestens umfassend drei Nadeln, wobei die distalen Enden der Nadeln einen Kreisbogen bilden, wobei dieser nicht auf die lokale Richtung der Nadeln im Bereich ihres distalen Endes senkrecht ist, wobei die betreffende Position der Nadeln jene ist, die sich in der Anschlagposition einnehmen, wenn sie gleitend auf dem Träger montiert sind.

## Claims

1. Injection device intended for injecting a product into an eye, or for electroporation, comprising:
- a needle in the form of an injection needle and/or of an invasive electrode (14₁, 14₂, 14₃; 14₁', 14₂', 14₃'),
- a support (12) on which the needle is fixed or which is able to guide the sliding of the needle along the axis of the said needle,
the device being **characterized in that** it comprises an identifying mark (50) fixed to the support and arranged along an arc of a circle of a radius greater than 5 mm and less than 8 mm, the length of said arc of a circle being less than 20 mm, so that it can be brought into contact with a determined bearing region (E) of the surface of the eye (0) before the needle (14₁, 14₂, 14₃; 14₁', 14₂', 14₃') makes any contact with the said surface, and then be kept in contact with the said bearing region during a phase in which the said needle penetrates through the said surface of the eye, the bearing region being at least part of the margin (E) of the cornea of the eye (0), the said penetration resulting from a rotation of the support (12) about the identifying mark (50) when the said needle is fixed on the support (12).

2. Device according to the preceding claim, in which the needle (14₁, 14₂, 14₃; 14₁', 14₂', 14₃') is configured in such a way that, throughout the phase during which the said needle is penetrating the eye, the local direction (D_{needle}) of the needle at the point (P₁) of penetration into the eye (O) forms, with the direction (*̅V̅*̅₁₄) of penetration at the said penetration point (P₁) an angle β (β₁, β₂, β₃) that is always smaller than a maximum divergence angle βₘₐₓ of 15°.

3. Device according to either one of the preceding claims, in which the needle is configured in such a way that its distal end (17₁) can reach the ciliary muscle of the eye (O) during the said needle penetration phase.

4. Device according to any one of the preceding claims, in which the needle is made at least in part from an electrically conducting material, the device comprising connecting means allowing the said needle to be electrically connected to an electric generator.

5. Device according to any one of the preceding claims, comprising support end-stop means able to limit the movement of the support during the penetration phase, the said end-stop means being configured in such a way as to limit the length of the said needle that can be driven into the eye, during the said penetration phase, to less than 20 mm.

6. Device according to the preceding claim, in which the end-stop means are configured in such a way as to limit the length of the said needle that be driven into the eye, during the said penetration phase, to less than 3 mm.

7. Device according to any one of the two immediately preceding claims, in which the end-stop means comprise a stop face (40) extending substantially in a spherical envelope, the radius of curvature of the said spherical envelope being greater than 9 mm and less than 14 mm.

8. Device according to Claim 7, in which the stop face (40) does not extend over more than one quadrant of a hemisphere.

9. Device according to either one of Claims 7 and 8, comprising several needles extending inside the said spherical envelope until they reach an identical depth.

10. Device of any one of Claims 5 to 9, in which the said end-stop means comprise a stop face extending along a spherical envelope (S) and in which the identifying mark is an identifying line in the form of an arc of a circle (F₅₀), the axis of said axis of a circle (Dcₒ) passing through the centre (C) of the said spherical envelope (S).

11. Device according to any one of the preceding claims, in which the point of insertion of the said needle into the said support is outside the virtual spherical surface a great circle of which includes the identifying mark.

12. Device according to any one of Claims 5 to 10, in which the said end-stop means comprise a stop face extending substantially along a spherical envelope (S), the identifying mark extends along an identifying line in the form of an arc of a circle (F₅₀), at least one needle penetrates the spherical envelope via a penetration point (Pᵢ) and, in a plane (Plᵢ) passing through the centre (C) of the said spherical envelope (S) via the said penetration point (Pᵢ) and intersecting the said arc of a circle (F₅₀) at right angles, the distance (lᵢ) between the said penetration point (Pᵢ) and the point (Qᵢ) on the said arc of a circle at which said plane (Plᵢ) intersects the said arc of a circle (F₅₀) is
(1) greater than 1 mm and less than 3 mm, the said needle extending inside the said spherical envelope as far as a depth (Δᵢ) greater than 0.5 mm and less than 1.5 mm; or
(2) greater than 2 mm and less than 4.5 mm, the said needle extending into the said spherical envelope as far as a depth (Δᵢ) greater than 6 mm and less than 15 mm.

13. Device according to any one of the preceding claims, comprising a set of electrodes made up of first (14₁, 14₂, 14₃; 14₁', 14₂', 14₃') and second (52) sets of electrodes intended to be electrically connected to first and second terminals, respectively, of an electric generator and in which the said needle or needles constitute invasive electrodes of the said first set of electrodes.

14. Device according to the preceding claim, in which the second set of electrodes has an electrically conducting coating, and in which said coating (52) at least partially covers a stop face (40).

15. Device according to any one of the preceding claims, in which the identifying mark is configured to bear over more than 5 mm of the corneal margin.

16. Device according to any one of the preceding claims, comprising at least three needles, the distal ends of the said needles forming an arc of a circle, this arc not being perpendicular to the local direction of the said needles at their distal end, the position of the said needles considered being the position that they occupy in the position of abutment when they are mounted with the ability to slide on the said support.
